# EUROPEAN PATENT APPLICATION

(11) **EP 4 400 064 A2**
(43) Date of publication of application: **17.07.2024**
(21) Application number: 23219674.1
(22) Date of filing: 22.12.2023
(51) Int. Cl.: A61B 18/14, A61M 25/10

(54) **RAPID DEPRESSURIZATION OF IRRIGATED BALLOON CATHETER**

(30) Priority: 23.12.2022 US 202218146110
(71) Applicant: Biosense Webster (Israel) Ltd., 2066717 Yokneam (IL)
(72) Inventor: GOVEA, Michael, Irvine, 92618 (US); RODRIGUEZ, Jasson, Irvine, 92618 (US)
(74) Representative: Carpmaels & Ransford LLP

(57) **Abstract**

The disclosed technology includes a fluid evacuation device for a balloon catheter including at least one spine having a curved proximal portion and a straight distal portion extending along a longitudinal axis, a first coupler coupled to the curved proximal portion of the spine to constrain at least one spine to move along the longitudinal axis, and a second coupler attached to the distal portion of the at least one spine so that in a first operational mode, the curved proximal portion of the at least one spine is expanded to a first outer profile and in a second operational mode, the curved proximal portion of the at least one spine is compressed to a smaller outer profile than the first outer profile such that the at least one spine moves the second coupler along the longitudinal axis.

## Description

### FIELD

The present invention relates generally to minimally invasive medical devices, and in particular balloon catheters, and further relates to, but not exclusively, irrigated balloon catheters.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

In some procedures, a catheter with one or more RF electrodes may be used to provide ablation within the cardiovascular system. The catheter may be inserted into a major vein or artery (e.g., the femoral artery) and then advanced to position the electrodes within the heart or in a cardiovascular structure adjacent to the heart (e.g., the pulmonary vein). The one or more electrodes may be placed in contact with cardiac tissue or other vascular tissue and then activated with RF energy to thereby ablate the contacted tissue. In some cases, the electrodes may be bipolar. In some other cases, a monopolar electrode may be used in conjunction with a ground pad or other reference electrode that is in contact with the patient. Irrigation may be used to draw heat from ablating components of an ablation catheter; and to prevent the formation of blood clots near the ablation site.

Examples of ablation catheters are described in U.S. Pat. No. 10,743,932, entitled "Integrated Ablation System using Catheter with Multiple Irrigation Lumens," issued August 18, 2020, the disclosure of which is incorporated by reference herein, in its entirety; U.S. Pat. No. 10,660,700, entitled "Irrigated Balloon Catheter with Flexible Circuit Electrode Assembly," issued May 26, 2020, the disclosure of which is incorporated by reference herein, in its entirety; and U.S. Pat. No. 10,130,422, entitled "Catheter with Soft Distal Tip for Mapping and Ablating Tubular Region," issued November 20, 2018, the disclosure of which is incorporated by reference herein, in its entirety.

Some catheter ablation procedures may be performed after using electrophysiology (EP) mapping to identify tissue regions that should be targeted for ablation. Such EP mapping may include the use of sensing electrodes on a catheter (e.g., the same catheter that is used to perform the ablation or a dedicated mapping catheter). Such sensing electrodes may monitor electrical signals emanating from conductive endocardial tissues to pinpoint the location of aberrant conductive tissue sites that are responsible for the arrhythmia. Examples of EP mapping systems and catheters are described in various references cited herein.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode probes was previously proposed in the patent literature. Examples of systems and devices configured for IRE ablation are disclosed in U.S. Patent Pub. Nos. 2021/0169550A1, 2021/0169567A1, 2021/0169568A1, 2021/0161592A1, 2021/0196372A1, 2021/0177503A1, and 2021/0186604A1, each of which are incorporated herein by reference.

Regions of cardiac tissue can be mapped by a catheter to identify the abnormal electrical signals. The same or different medical probe can be used to perform ablation. Using a therapeutic balloon catheter requires inflation (for ablation or mapping) and deflation (for re-sheathing and positioning) of the balloon. While inflation is accomplished via external fluid pump, deflation is a mostly passive activity, relying on fluid escape from the balloon. This fluid evacuation can take a significant duration of time (e.g., greater than 30 seconds) and is limited by the size and total surface area of the existing balloon irrigation holes. As will be appreciated, waiting a significant duration of time to deflate the balloon can extend surgery times and, consequently, extend patient recovery time. What is needed, therefore, are systems and methods of rapidly deflating a therapeutic balloon of a balloon catheter.

### SUMMARY

There is provided, in accordance with an embodiment of the present invention, a fluid evacuation device for a balloon catheter including at least one spine having a curved proximal portion and a straight distal portion extending along a longitudinal axis, a first coupler coupled to the curved proximal portion of the spine to constrain at least one spine to move along the longitudinal axis, and a second coupler attached to the distal portion of the at least one spine so that in a first operational mode, the curved proximal portion of the at least one spine is expanded to a first outer profile and in a second operational mode, the curved proximal portion of the at least one spine is compressed to a smaller outer profile than the first outer profile such that the at least one spine moves the second coupler along the longitudinal axis.

The fluid evacuation device can further include a sheath. At least one spine can be one of a plurality of spines forming a cage with each of the plurality of spines having a curved proximal portion and a straight distal portion. The curved proximal portion can be configured to transition from the first outer profile to the smaller outer profile responsive to a retraction of the cage into the sheath.

The fluid evacuation device can further include a tubular shaft disposed on the longitudinal axis and a sleeve disposed radially around the plurality of spines between the curved proximal portion and the straight distal portion. The sleeve can be attached to the tubular shaft and configured to constrain the curved proximal portion to move relative to the first coupler along the longitudinal axis with respect to the tubular shaft. The tubular shaft can include a guidewire lumen.

The fluid evacuation device can further include a sleeve disposed radially around the plurality of spines between the proximal portion and the distal portion, the sleeve being attached to the cage.

The disclosed technology includes a fluid evacuation device for a balloon catheter. The fluid evacuation device can include a tubular shaft extending along a longitudinal axis from a first end to a second end, the tubular shaft including at least one fluid port in fluid communication with a fluid supply to allow fluid flow in and out of the tubular shaft, at least one spine having a curved proximal portion and a straight distal portion extending along the longitudinal axis, a first coupler attached to the proximal portion of the at least one spine, and a second coupler attached to the distal portion of the at least one spine, the second coupler configured to move with respect to the tubular shaft so that movement of the second coupler in a first direction prevents fluid from flowing through the at least one fluid port of the tubular shaft in a first position and movement of the second coupler in a second direction permits fluid to flow through the at least one fluid port of the tubular shaft in a second position.

The at least one spine can be configured to slide the second coupler between the first position and the second position.

The at least one spine can be one of a plurality of spines forming a cage, each of the plurality of spines having a curved proximal portion and a straight distal portion, the curved proximal portion configured to straighten responsive to a retraction of the cage into a sheath.

The fluid evacuation device can further include a sleeve disposed radially around the plurality of spines between the proximal portion and the distal portion. The sleeve can be attached to the tubular shaft and configured to constrain the curved proximal portion to move relative to the first coupler along the longitudinal axis with respect to the tubular shaft.

The fluid evacuation device can further include a sleeve disposed radially around the plurality of spines between the proximal portion and the distal portion, the sleeve attached to the cage.

The second coupler can be configured to rotate between the first position and the second position.

The at least one spine can be configured such that a retraction of the at least one spine into a sheath rotates the second coupler from the first position to the second position.

The second coupler can include a hole configured to align with an evacuation port disposed on the tubular shaft when in the second position.

The disclosed technology includes a balloon catheter. The balloon catheter can include a balloon defining an interior volume configured to be inflated by filling with a fluid. The balloon can include a fluid evacuation device including a tubular shaft extending along a longitudinal axis from a first end to a second end, the tubular shaft including at least one fluid port in fluid communication with a fluid supply to allow fluid flow in and out of the tubular shaft, at least one spine having a curved proximal portion and a straight distal portion extending along a longitudinal axis, a first coupler coupled to the proximal portion of the at least one spine, and a second coupler attached to the distal portion of the at least one spine, the second coupler being configured to move with respect to the tubular shaft so that movement of the second coupler prevents fluid from flowing through the at least one fluid port of the tubular shaft in a first position and movement of the second coupler permits fluid to flow through the at least one fluid port of the tubular shaft in a second position.

The spine can be configured to slide the second coupler from the first position to the second position.

The balloon catheter can further include a sheath configured to deliver the balloon. The sheath can include a distal opening configured to deploy the balloon and receive the balloon upon a retraction of the balloon into the distal opening, the retraction applying a force to the spine and the balloon, and the spine being one of a plurality of spines forming a cage, each of the plurality of spines having a curved proximal portion and a straight distal portion, the curved portion being configured to straighten responsive to a retraction of the cage into a sheath.

The cage can be configured to slide the second coupler along a tubular shaft from the first position to the second position in response to the force, and the force can be configured to forcibly deflate the balloon. Additionally, the cage can also be configured to rotate the second coupler from the first position to the second position in response to the force, and the force can be configured to forcibly deflate the balloon.

The balloon can further include a plurality of irrigation ports configured to deliver fluid.

### BRIEF DESCRIPTION OF THE DRAWINGS

FIG. 1 is a schematic pictorial illustration of a medical system including a balloon catheter, in accordance with an embodiment of the present invention;
FIG. 2 shows a top view of a balloon of a balloon catheter in an inflated state, in accordance with an embodiment of the present invention;
FIG. 3A shows a side view of a balloon catheter in an inflated state, in accordance with an embodiment of the present invention;
FIG. 3B shows a side view of a balloon catheter in a deflated state, in accordance with an embodiment of the present invention;
FIG. 4 shows a sectional view of a balloon catheter showing a side view of fluid evacuation device in a first operational mode, in accordance with the disclosed technology;
FIG. 5 shows a sectional view of a balloon catheter showing a side view a fluid evacuation device in a second operational mode, in accordance with the disclosed technology;
FIG. 6 shows a perspective view of a fluid evacuation device, in accordance with the disclosed technology;
FIG. 7 shows a front view of a sleeve of the fluid evacuation device, in accordance with the disclosed technology;
FIG. 8A shows a side view of a fluid evacuation device of a balloon catheter in a first operational mode with a second coupler disposed in a first position along a tubular shaft, in accordance with the disclosed technology, in accordance with the disclosed technology;
FIG. 8B shows a side view of a fluid evacuation device of a balloon catheter in a second operational mode with a second coupler disposed in a second position along a tubular shaft, in accordance with the disclosed technology, in accordance with the disclosed technology;
FIG. 9 shows a side view of a fluid evacuation device of a balloon catheter configured to rotate between a first position and a second position, in accordance with the disclosed technology;
FIG. 10A shows a fluid evacuation device of a balloon catheter in a first operational mode with a second coupler disposed in a first position along a tubular shaft, in accordance with the disclosed technology;
FIG. 10B shows a fluid evacuation device of a balloon catheter in a second operational mode with a second coupler disposed in a second position along a tubular shaft, in accordance with the disclosed technology; and
FIG. 11 is a method flow chart for a method for performing a medical operation, in accordance with the disclosed technology.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, reference to tissue, vasculature, or organs of a "patient," "host," "user," and "subject" can be that of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells. For example, by utilizing thermal energy, such as radio frequency (RF) ablation, or non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to thermal or non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The present disclosure is related to systems, method or uses and devices for ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for RF ablation, cryoablation, and/or irreversible electroporation (IRE). IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

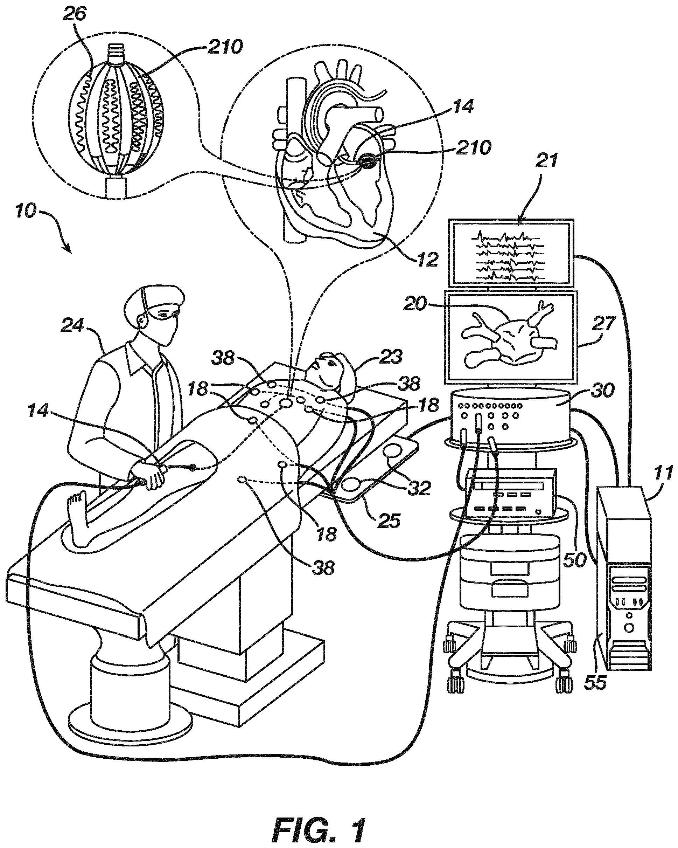

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 brings a distal tip of catheter 14 (i.e., a balloon catheter 200 in this case, the distal tip of which is sometimes referred to herein as balloon 210) into contact with the heart wall for sensing a target site in heart 12. For ablation, physician 24 would similarly bring a distal end of an ablation catheter to a target site for ablating.

Catheter 14 is an exemplary catheter that includes one and preferably multiple electrodes 26 optionally distributed over balloon 210 and configured to sense the IEGM signals. Catheter 14 may additionally include a position sensor 29 embedded in or near balloon 210 for tracking position and orientation of balloon 210. Optionally and preferably, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. Real time position of balloon 210 of catheter 14 may be tracked based on magnetic fields generated with location pad 25 and sensed by magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes 26 at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31A Technology Drive, Irvine, CA 92618.

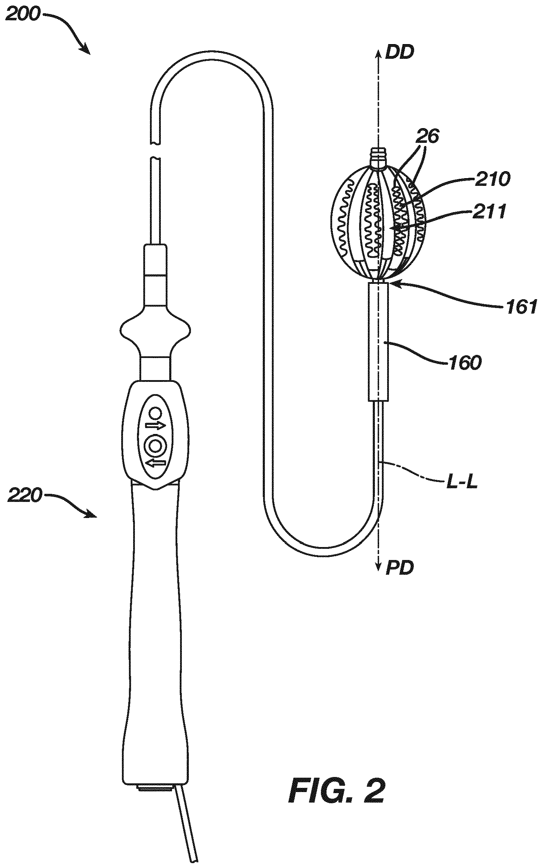

FIG. 2 shows a balloon 210 of a balloon catheter 200 in an inflated state, in accordance with an embodiment of the present invention. The balloon 210 is configured to inflate with a fluid to allow electrodes 26 disposed on the balloon 210 to contact target tissue such as that of heart 12. The fluid can include, for example, saline or other biocompatible fluids. Balloon catheter 200 includes an interior volume 211 which is configured to hold the fluid, and interior volume 211 varies depending on whether the balloon 210 is in an inflated state or in a deflated state. The balloon catheter 200 can include a handle 220 that allows physician 24 to manipulate the distal end of balloon catheter 200. The handle 220 can be coupled to an external fluid pump or other fluid sources as would be appreciated by one of skill in the pertinent art, and handle 220 can include actuators configured to allow physician 24 to selectively inflate balloon 210 with the fluid.

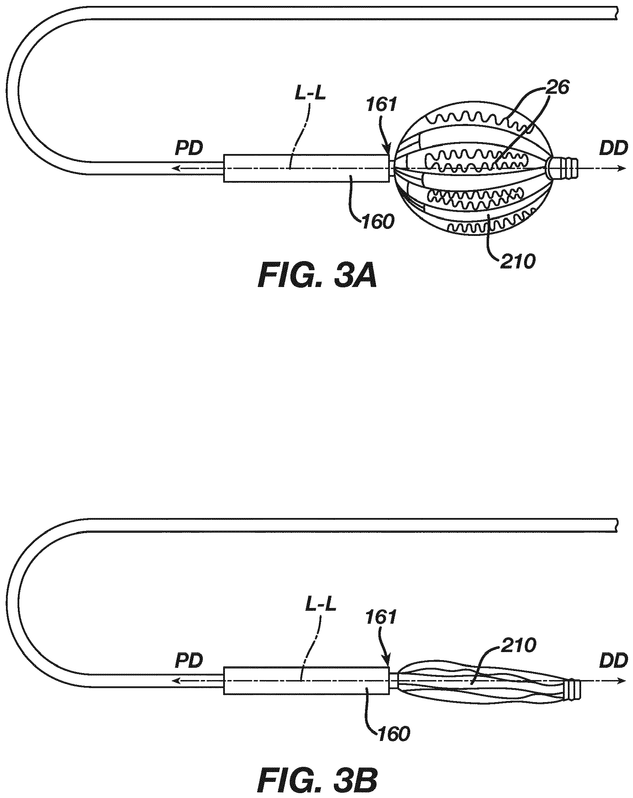

The balloon 210 can define an interior volume 211 configured to be inflated by filling the balloon 210 with a fluid such as, for example but not limitation, saline. For example, as shown in FIGs. 3A and 3B, the interior volume 211 of the balloon 210 can be inflated or deflated by the fluid. As shown in FIG. 3A, the interior volume 211 can be filled with a fluid to transition to an inflated state. In contrast, the fluid can be evacuated from the interior volume 211 to transition the balloon 210 to a deflated state (FIG. 3B). As will be appreciated, the balloon can be in the deflated state when being inserted into an organ (e.g., heart 12) or retracted into sheath 160 and then inflated when removed from the sheath 160 for performing mapping or ablation of tissue.

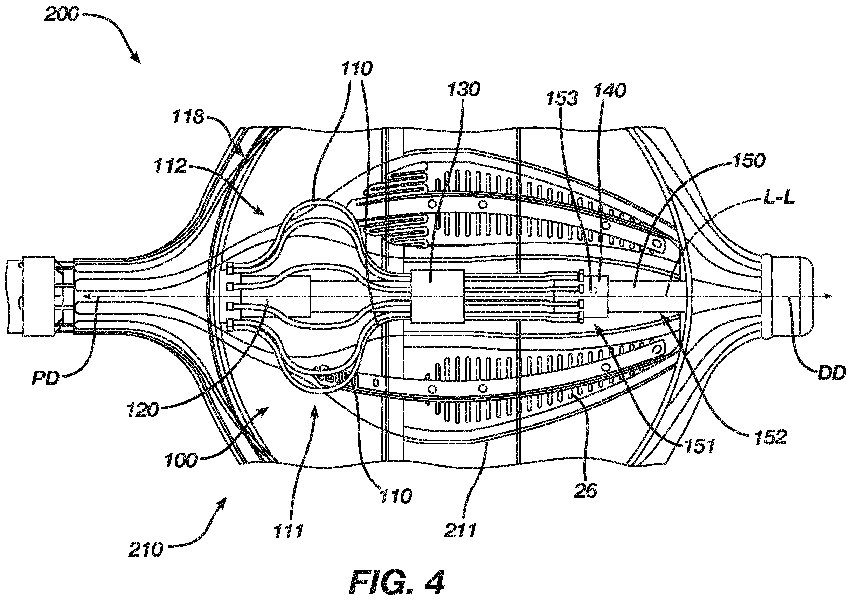

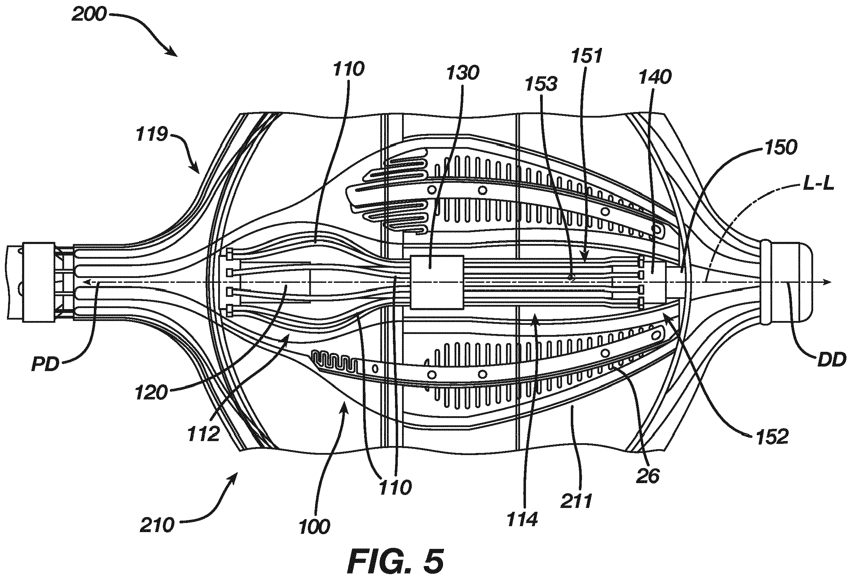

FIGs. 4 and 5 show sectional views of the balloon 210. The balloon 210 can include a fluid evacuation device 100 disposed in the interior volume 211 including a tubular shaft 150 extending along a longitudinal axis L-L from a first end (for example, in the proximal direction PD) to a second end (for example, in the distal direction DD). The tubular shaft 150 can include at least one fluid port 153 in fluid communication with a fluid supply to allow fluid flow in and out of the tubular shaft 150. The fluid evacuation device can include at least one spine 110 having a curved proximal portion 112 and a straight distal portion 114 extending along a longitudinal axis L-L, a first coupler 120 coupled to the proximal portion 112 of the at least one spine 110, and a second coupler 140 attached to the distal portion 114 of the at least one spine 110, the second coupler 140 being configured to move with respect to the tubular shaft 150 so that movement of the second coupler 140, for example proximal direction PD, prevents fluid from flowing through the at least one fluid port 153 of the tubular shaft 150 in a first position 151, and movement of the second coupler 140, for example distal direction DD permits fluid to flow through the at least one fluid port 153 of the tubular shaft 150 in a second position 152. In some examples, the at least one spine 110 configured to slide the second coupler 140 between the first position 151 and the second position 152. The tubular shaft 150 can include a lumen therethrough through which fluid can flow from the interior of the balloon 210 to the exterior of the balloon 210. The spine 110 can be configured to slide the second coupler 140 from the first position 151 to the second position 152. The balloon 210 can include a plurality of irrigation ports configured to deliver the fluid to tissue or the electrodes 26 to cool the electrodes 26 and/or tissue proximate the electrodes 26 during ablation. In some examples, the plurality of irrigation ports can be configured to have a total outlet area less than that of fluid port 153 such that the balloon 210 can remain inflated while the fluid evacuation device 100 is in the first operational mode 118 (FIG. 4) while fluid weeps out from interior volume 211 of balloon 210 and the balloon 210 can be deflated while fluid evacuation device 100 is in second operational mode 119 (FIG. 5). In some examples, the balloon 210 when in the first operational mode 118 can be continuously flushed with fluid in order to cool the electrodes 26 of the balloon catheter 200.

As shown in FIG. 2 and FIGs. 3A and 3B, the balloon catheter 200 can further include a sheath 160 configured to receive the balloon 210 and facilitate delivery of the balloon 210 to an organ. The sheath 160 can include a distal opening 161 (as shown in FIGs. 2, 3A, and 3B) configured to deploy the balloon 210 and receive the balloon 210 upon a retraction of the balloon 210 into the distal opening 161. When the balloon 210 is out of the distal opening 161 as in FIG. 4, the sheath 160 does not apply a force to the spine 110 and the fluid evacuation device 100 is in the first operational mode 118 (FIG. 4). When the balloon 210 is retracted into the distal opening 161, as in FIG. 5, the retraction results in the sheath 160 applying a force to the spine 110 and moving the second coupler 140 distally, thereby transitioning the fluid evacuation device 100 to the second operational mode.

In some examples, the curved proximal portion 112 of the spine 110 can be shaped as a symmetrical bow approximating a normal bell curve such that the spine 110 resists retraction into the sheath 160 smoothly across the length of the spine 110. In other examples, the curved proximal portion 112 can include a steepened proximal portion approximating a proximally skewed bell curve such that the spine 110 resists retraction into the sheath 160 unevenly, namely that the force required to result in the retraction of the proximal half of the proximal portion 112 of the spine is between about two and about ten times greater than the force required to result in the retraction of the distal half of the proximal portion 112 of the spine into the sheath 160.

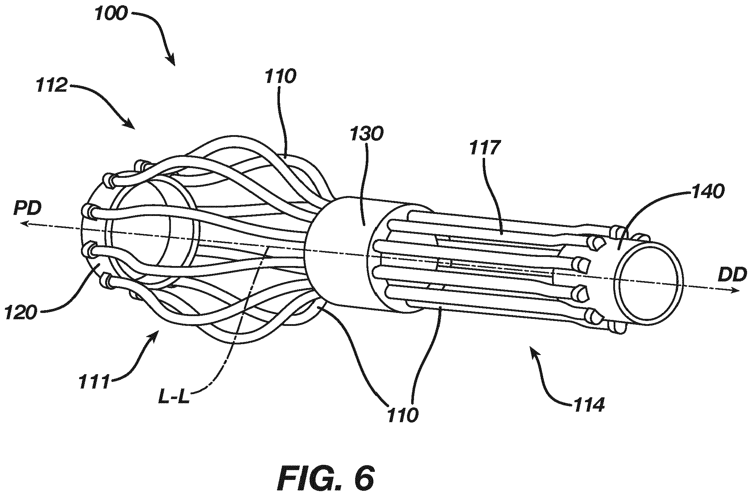

FIG. 6 shows an example fluid evacuation device, in accordance with the disclosed technology. The fluid evacuation device 100 for a balloon catheter 200 can include at least one spine 110 having a curved proximal portion 112 and a straight distal portion 114 extending along a longitudinal axis L-L, a first coupler 120 coupled to the curved proximal portion 112 of the spine 110 to constrain at least one spine 110 to move along the longitudinal axis L-L, and a second coupler 140 attached to the distal portion 114 of the at least one spine 110 so that in a first operational mode 118, the curved proximal portion 112 of the at least one spine 110 is expanded to a first outer profile 116 and in a second operational mode 119, the curved proximal portion 112 of the at least one spine 110 is compressed to a smaller outer profile 117 than the first outer profile 116 such that the at least one spine 110 moves the second coupler 140 along the longitudinal axis L-L.

The spine 110 can be a single spine or one of a plurality of spines 110 forming a cage 111. Each spine 110 can have a curved proximal portion 112 and a straight distal portion 114. The curved proximal portion 112 can be configured to straighten responsive to a retraction of the cage 111 into the sheath 160. In other words, because the spine 110 bows radially outward, the spine 110 is forced to bend inwardly as the balloon 210 is retracted into the sheath 160 as a result of the spine 110 bowing radially outward a greater distance from the longitudinal axis L-L than a distance of the inner diameter of the sheath 160 from the longitudinal axis L-L. As the spine 110 is bent inwardly, the spine 110 (or cage 111) can be configured to slide the second coupler 140 along a tubular shaft 150 from the first position 151 to the second position 152 in response to the force. As the second coupler 140 is moved to the second position by the force from the balloon 210 contacting the sheath 160, the balloon 210 can be deflated as the fluid escapes through the fluid port 153 and out through the lumen formed in the tubular shaft 150.

Furthermore, at least the curved proximal portion 112 of the spines 110 can be biased such that the spines 110 naturally bow radially outward from the longitudinal axis L-L when the balloon 210 is pushed out from the sheath 160. In this way, the fluid evacuation device 100 can be configured to remain in the first operational mode 118 unless the balloon 210 and fluid evacuation device 100 are retracted into the sheath 160.

Another example fluid evacuation device 100 for a balloon catheter 200 can include a tubular shaft 150 extending along a longitudinal axis L-L from a first end (for example, in the proximal direction PD) to a second end (for example, in the distal direction DD). The tubular shaft 150 can include at least one fluid port 153 in fluid communication with a fluid supply to allow fluid flow in and out of the tubular shaft 150, at least one spine 110 having a curved proximal portion 112 and a straight distal portion 114 extending along the longitudinal axis L-L, a first coupler 120 attached to the proximal portion 112 of the at least one spine 110, and a second coupler 140 attached to the distal portion 114 of the at least one spine 110, the second coupler 140 configured to move with respect to the tubular shaft 150 so that movement of the second coupler 140 in a first direction, for example proximal direction PD prevents fluid from flowing through the at least one fluid port 153 of the tubular shaft 150 in a first position 151, and movement of the second coupler 140 in a second direction, for example distal direction DD, permits fluid to flow through the at least one fluid port 153 of the tubular shaft 150 in a second position 152. In some examples, the at least one spine 110 configured to slide the second coupler 140 between the first position 151 and the second position 152.

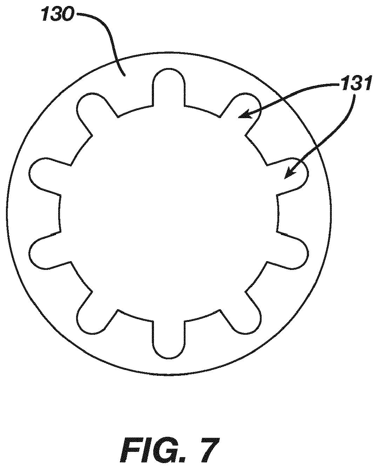

As shown in FIG. 7, the sleeve 130 can include a plurality of channels 131 through which the plurality of spines 110 pass can pass. The channels 131 can be configured to permit the spines 110 to move axially along the longitudinal axis L-L but prevent the spines 110 from bowing radially outwardly or from rotating about the longitudinal axis L-L.

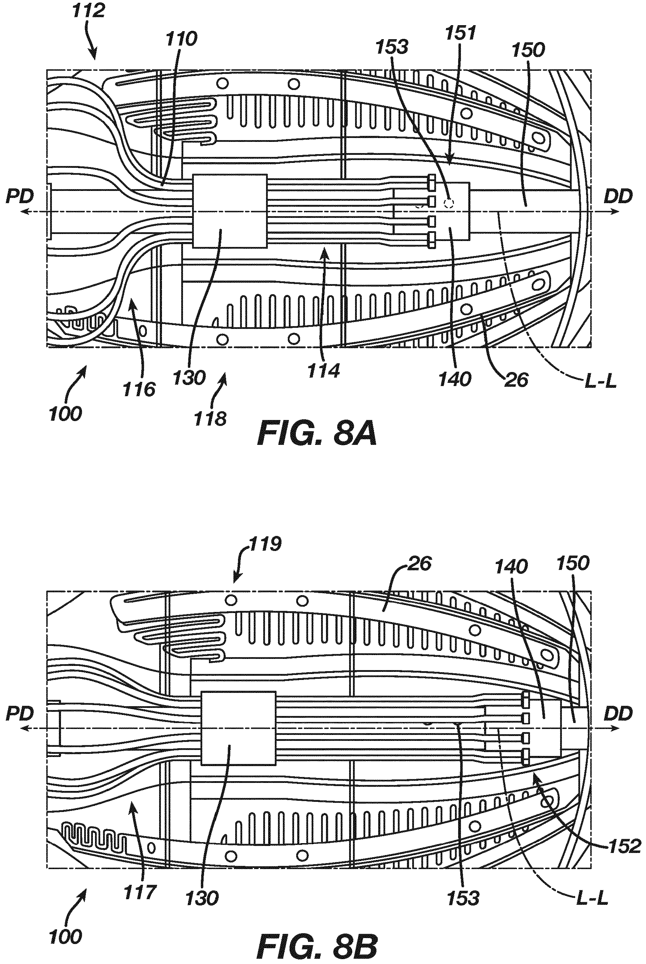

FIG. 8A is a detail view showing the second coupler 140 in the first position 151. When the second coupler 140 is in the first position 151 (i.e., the fluid evacuation device 100 is in the first operational mode 118), the fluid port 153 is covered by the second coupler 140. In this way, the second coupler 140 seals off the fluid communication between the interior volume 211 and the fluid port 153 thereby preventing leakage of the fluid through the fluid port 153 and helping to maintain inflation of the balloon 210.

FIG. 8B shows the second coupler 140 in the second position 152. When the second coupler 140 is in the second position 152 (i.e., the fluid evacuation device 100 is in the second operational mode 119), the fluid port 153 is unobstructed or otherwise not covered by the second coupler 140 and fluid within the interior volume 211 of the balloon 210 is permitted to escape through the fluid port 153, thereby permitting the balloon 210 to deflate.

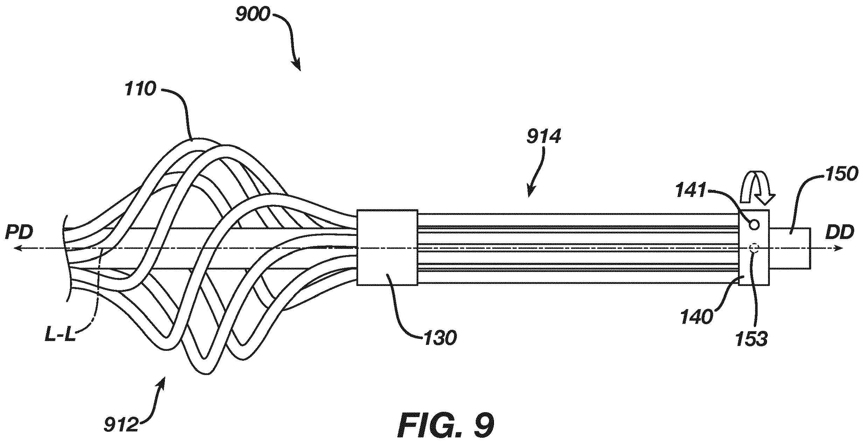

Alternatively, as shown in FIG. 9 the cage 111 can be configured to rotate the second coupler 140 from the first position 151 to the second position 152 in response to the force. As before, when the second coupler 140 is moved to the second position by the force from the balloon 210 contacting the sheath 160, the balloon 210 can be deflated as the fluid escapes through the fluid port 153 and out through the lumen formed in the tubular shaft 150. The at least one spine 110 can be configured such that a retraction of the at least one spine 110 into a sheath 160 rotates the second coupler 140 from the first position 151 to the second position 152. For example, a proximal end 912 of the spine 110 can be pitched helically around the tubular shaft 150 so that the spine 110 rotates upon the retraction, thus causing a rotation of the second coupler 140 which is attached to the at least one spine 110 at a distal end. In some examples, the second coupler 140 can include a hole 141 configured to align with an evacuation port 153 disposed on the tubular shaft 150 when in the second position 152. In some examples, the helical pitch of the spine 110 can be shaped as a helically pitched symmetrical bow approximating a normal bell curve such that the spine 110 resists retraction into the sheath 160 evenly across the length of the spine 110. In another example, the curved proximal portion 112 can include a helically pitched steepened proximal portion approximating a helically pitched proximally skewed bell curve such that the spine 110 resists retraction into the sheath unevenly, namely that the force required to result in the first half of the rotation is between about two and about ten times greater than the force required to result in the second half of the rotation. In some examples, such as that shown in FIG. 9, the number of spines in the helically pitched proximal portion 112 of the cage 111 can be different than the number of spines than the distal portion 114. In other examples, the number of spines can be the same throughout the length of the cage 111.

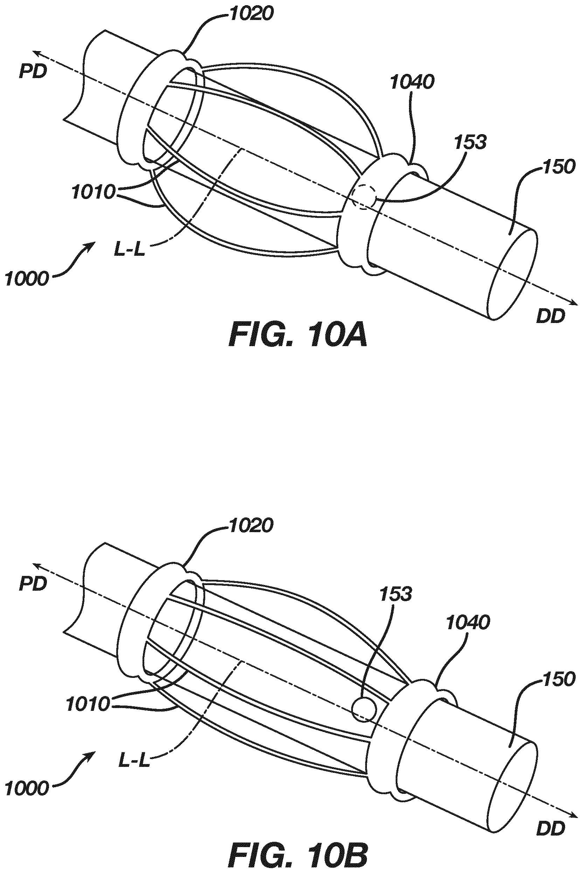

FIG. 10A and FIG. 10B show a fluid evacuation device 1000 of a balloon catheter 200 in a first operational mode with a second coupler 1040 disposed in a first position 151 along a tubular shaft 150, in accordance with the disclosed technology. In the example provided in FIGs. 10A and 10B, the spine 1010 does not have a straight distal portion but rather a continuous curved portion extending from the first coupler 1020 to the second coupler 1040. In this example, the straightening of the curved spine 110 causes a translation of the second coupler 1040 along the longitudinal axis L-L. In the first position 151, the fluid port 153 is blocked.

In any of the examples disclosed herein, the fluid port 153 can be sized such that the balloon 210 can be substantially completely evacuated of fluid within about 3 seconds of the retraction. In any of the examples disclosed herein, the fluid port 153 can be sized such that the balloon 210 can be substantially completely evacuated of fluid within less than 1 second of the retraction. In any of the examples disclosed herein, the fluid port 153 can be sized such that the balloon 210 can be substantially completely evacuated of fluid within about 5 to about 10 seconds of the retraction. Additionally, more fluid ports can be added to the tubular lumen 150 to allow the fluid to be evacuated faster. Furthermore, the time taken for the fluid to evacuate can be modulated based on the force of retraction applied by the user, with greater forces resulting in lower times to evacuate compared to lower forces.

In any of the examples disclosed herein, spine 110 may have elliptical (e.g., circular) or rectangular (that may appear to be flat) cross-sections and can be a flexible, resilient material (e.g., a shape-memory alloy such as nickel-titanium, also known as Nitinol). Further, the spine can include a material selected from a group including of nitinol, cobalt chromium, stainless steel, titanium. Alternatively, or in addition, the spine 110 can include a polymer. The spine 110 can have a circular cross section. The spine 110 can also be a ribbon with a substantially flat, rectangular cross section so as to minimize the profile of the fluid evacuation device 100. Furthermore, the cage 111 can be formed by cutting spines from a tube, such as a nitinol tube.

In some examples, the sleeve 130 can be attached to the tubular shaft 150 and configured to constrain the curved proximal portion 112 to move relative to the first coupler 120 along the longitudinal axis L-L with respect to the tubular shaft 150. In some examples, the sleeve 130 can function to constrain the spines 110 such that retracting the curved proximal portion 112 into the sheath 160 translates more directly to linear motion of the second coupler 140.

Alternatively, in some examples, the fluid evacuation device 100 the sleeve 130 can be disposed radially around the plurality of spines 110 between the proximal portion 112 and the distal portion 114 and attached to the cage 111. In this configuration, the sleeve 130 keeps the portion of the spines 110 distal to the sleeve 130 straight, which ensures that retraction of the cage 111 into the sheath 160 causes the curved proximal portion 112 to straighten and translate the second coupler 140 distally.

In some examples, the tubular shaft 150 can be a guidewire lumen, or can be part of a guidewire lumen. The balloon catheter 200 can be navigated to the target anatomy by the guidewire. The guidewire lumen can be sized to allow fluid evacuation in the times previously described.

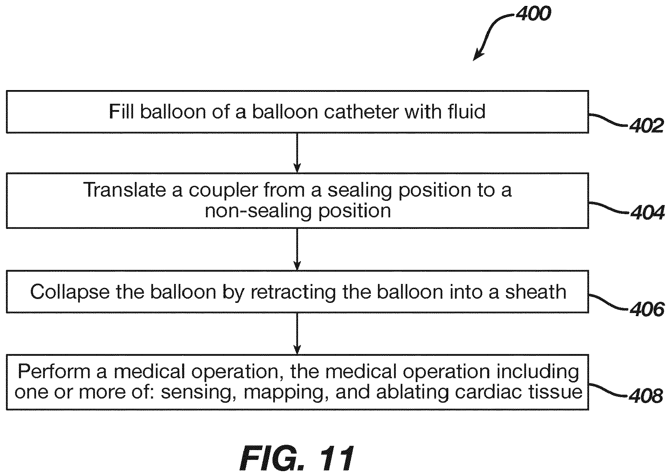

FIG. 11 is a method flow chart for a method 400 for performing a medical operation, in accordance with the disclosed technology. Method 400 can include filling a balloon of a balloon catheter with fluid (step 402) causing a fluid to evacuate through an irrigation port of a balloon catheter. The method 400 can include translating a coupler from a sealing position to a non-sealing position (step 404) permitting fluid to evacuate from the balloon. The method 400 can include and collapsing the balloon by retracting the balloon into a sheath (step 406).

In some examples, translating the coupler can include straightening a spine by retracting a curved proximal portion of the spine proximally into the sheath, the spine being in fixed communication with the coupler. Retracting the curved proximal portion into sheath can be part of retracting balloon into sheath. Alternatively, or additionally, retracting curved proximal portion can include pulling a pull wire attached to the fluid evacuation device at the proximal portion. The pull wire can be operatively coupled to an actuator disposed on handle of the balloon catheter such that pulling the pull wire can be performed by actuating the actuator.

In some examples, translating the coupler can include retracting the spine into the sheath. The spine can be configured to rotate the coupler from the sealing position to the non-sealing position upon the retracting.

In some examples, the method can further include performing a medical operation (step 408), the medical operation including one or more of: sensing, mapping, and ablating cardiac tissue.

As will be appreciated, the method 400 just described can be varied in accordance with the various elements and implementations described herein. That is, methods in accordance with the disclosed technology can include all or some of the steps described above and/or can include additional steps not expressly disclosed above. Further, methods in accordance with the disclosed technology can include some, but not all, of a particular step described above. Further still, various methods described herein can be combined in full or in part. That is, methods in accordance with the disclosed technology can include at least some elements or steps of a first method and at least some elements or steps of a second method.

The disclosed technology described herein can be further understood according to the following clauses:
Clause 1: A fluid evacuation device for a balloon catheter comprising: at least one spine comprising a curved proximal portion and a straight distal portion extending along a longitudinal axis; a first coupler coupled to the curved proximal portion of the spine to constrain at least one spine to move along the longitudinal axis; and a second coupler attached to the distal portion of the at least one spine so that in: (a) a first operational mode, the curved proximal portion of the at least one spine is expanded to a first outer profile; and (b) in a second operational mode, the curved proximal portion of the at least one spine is compressed to a smaller outer profile than the first outer profile such that the at least one spine moves the second coupler along the longitudinal axis.
Clause 2: The fluid evacuation device of Clause 1, further comprising a sheath, the at least one spine being one of a plurality of spines forming a cage, each of the plurality of spines having a curved proximal portion and a straight distal portion, the curved proximal portion configured to transition from the first outer profile to the smaller outer profile responsive to a retraction of the cage into the sheath.
Clause 3: The fluid evacuation device of Clause 2, further comprising a tubular shaft disposed on the longitudinal axis and a sleeve disposed radially around the plurality of spines between the curved proximal portion and the straight distal portion, the sleeve attached to the tubular shaft and configured to constrain the curved proximal portion to move relative to the first coupler along the longitudinal axis with respect to the tubular shaft.
Clause 4: The fluid evacuation device of clause 3, wherein the tubular shaft comprises a guidewire lumen.
Clause 5: The fluid evacuation device of Clause 2, further comprising a sleeve disposed radially around the plurality of spines between the proximal portion and the distal portion, the sleeve attached to the cage.
Clause 6: A fluid evacuation device for a balloon catheter, the fluid evacuation device comprising: a tubular shaft extending along a longitudinal axis from a first end to a second end, the tubular shaft including at least one fluid port in fluid communication with a fluid supply to allow fluid flow in and out of the tubular shaft; at least one spine comprising a curved proximal portion and a straight distal portion extending along the longitudinal axis; a first coupler attached to the proximal portion of the at least one spine; and a second coupler attached to the distal portion of the at least one spine, the second coupler configured to move with respect to the tubular shaft so that movement of the second coupler in a first direction prevents fluid from flowing through the at least one fluid port of the tubular shaft in a first position; and movement of the second coupler in a second direction permits fluid to flow through the at least one fluid port of the tubular shaft in a second position.
Clause 7: The fluid evacuation device of Clause 6, the at least one spine configured to slide the second coupler between the first position and the second position.
Clause 8: The fluid evacuation device according to any of Clauses 6-7, the at least one spine being one of a plurality of spines forming a cage, each of the plurality of spines having a curved proximal portion and a straight distal portion, the curved proximal portion configured to straighten responsive to a retraction of the cage into a sheath.
Clause 9: The fluid evacuation device of Clause 8, further comprising a sleeve disposed radially around the plurality of spines between the proximal portion and the distal portion, the sleeve attached to the tubular shaft and configured to constrain the curved proximal portion to move relative to the first coupler along the longitudinal axis with respect to the tubular shaft.
Clause 10: The fluid evacuation device of Clause 8, further comprising a sleeve disposed radially around the plurality of spines between the proximal portion and the distal portion, the sleeve attached to the cage.
Clause 11: The fluid evacuation device according to any of Clauses 6-10, wherein the tubular shaft comprises a guidewire lumen.
Clause 12: The fluid evacuation device of Clause 6, the second coupler configured to rotate between the first position and the second position.
Clause 13: The fluid evacuation device of Clause 12, the at least one spine configured such that a retraction of the at least one spine into a sheath rotates the second coupler from the first position to the second position.
Clause 14: The fluid evacuation device of Clause 13, the second coupler comprising a hole configured to align with an evacuation port disposed on the tubular shaft when in the second position.
Clause 15: A balloon catheter comprising: a balloon defining an interior volume configured to be inflated by filling with a fluid, the balloon comprising: a fluid evacuation device comprising: a tubular shaft extending along a longitudinal axis from a first end to a second end, the tubular shaft including at least one fluid port in fluid communication with a fluid supply to allow fluid flow in and out of the tubular shaft; at least one spine comprising a curved proximal portion and a straight distal portion extending along a longitudinal axis; a first coupler coupled to the proximal portion of the at least one spine; and a second coupler attached to the distal portion of the at least one spine, the second coupler configured to move with respect to the tubular shaft so that movement of the second coupler prevents fluid from flowing through the at least one fluid port of the tubular shaft in a first position; and movement of the second coupler permits fluid to flow through the at least one fluid port of the tubular shaft in a second position.
Clause 16: The balloon catheter of Clause 15, the spine configured to slide the second coupler from the first position to the second position.
Clause 17: The balloon catheter of Clause 16 further comprising a sheath configured to deliver the balloon, the sheath comprising a distal opening configured to deploy the balloon and receive the balloon upon a retraction of the balloon into the distal opening, the retraction comprising applying a force to the spine and the balloon, and the spine being one of a plurality of spines forming a cage, each of the plurality of spines having a curved proximal portion and a straight distal portion, the curved portion configured to straighten responsive to a retraction of the cage into a sheath.
Clause 18: The balloon catheter of Clause 17, the cage configured to slide the second coupler along a tubular shaft from the first position to the second position in response to the force, and the force configured to forcibly deflate the balloon.
Clause 19: The balloon catheter of Clause 17, the cage configured to rotate the second coupler from the first position to the second position in response to the force, and the force configured to forcibly deflate the balloon.
Clause 20: The balloon catheter according to any of Clauses 15-19, the balloon comprising a plurality of irrigation ports configured to deliver the fluid.
Clause 21: A method comprising: filling a balloon of a balloon catheter with fluid; and evacuating the fluid through an evacuation port of the balloon catheter, comprising: translating a coupler from a sealing position to an open position; and retracting the balloon into a sheath.
Clause 22: The method of Clause 21, the translating the coupler comprising straightening a spine by retracting a curved proximal portion of the spine proximally into the sheath, the spine in fixed communication with the coupler.
Clause 23: The method of Clause 21, the translating the coupler comprising retracting the spine into the sheath, the spine configured to rotate the coupler from the sealing position to the open position upon the retracting.
Clause 24: The method of Clause 21, further comprising performing a medical operation, the medical operation comprising one or more of: sensing, mapping, and ablating cardiac tissue.

The embodiments described above are cited by way of example, and the present invention is not limited by what has been particularly shown and described hereinabove. Rather, the scope of the invention includes both combinations and sub combinations of the various features described hereinabove, as well as variations and modifications thereof which would occur to persons skilled in the art upon reading the foregoing description and which are not disclosed in the prior art.

## Claims

1. A fluid evacuation device for a balloon catheter comprising:
at least one spine comprising a curved proximal portion and a straight distal portion extending along a longitudinal axis;
a first coupler coupled to the curved proximal portion of the spine to constrain at least one spine to move along the longitudinal axis; and
a second coupler attached to the distal portion of the at least one spine so that:
(a) in a first operational mode, the curved proximal portion of the at least one spine is expanded to a first outer profile; and
(b) in a second operational mode, the curved proximal portion of the at least one spine is compressed to a smaller outer profile than the first outer profile such that the at least one spine moves the second coupler along the longitudinal axis.

2. The fluid evacuation device of claim 1, further comprising a sheath, the at least one spine being one of a plurality of spines forming a cage, each of the plurality of spines having a curved proximal portion and a straight distal portion, the curved proximal portion configured to transition from the first outer profile to the smaller outer profile responsive to a retraction of the cage into the sheath.

3. The fluid evacuation device of claim 2, further comprising:
a tubular shaft disposed on the longitudinal axis; and
a sleeve disposed radially around the plurality of spines between the curved proximal portion and the straight distal portion, the sleeve attached to the tubular shaft and configured to constrain the curved proximal portion to move relative to the first coupler along the longitudinal axis with respect to the tubular shaft.

4. The fluid evacuation device of claim 3, wherein the tubular shaft comprises a guidewire lumen.

5. The fluid evacuation device of claim 2, further comprising a sleeve disposed radially around the plurality of spines between the proximal portion and the distal portion, the sleeve attached to the cage.

6. A fluid evacuation device for a balloon catheter, the fluid evacuation device comprising:
a tubular shaft extending along a longitudinal axis from a first end to a second end, the tubular shaft including at least one fluid port in fluid communication with a fluid supply to allow fluid flow in and out of the tubular shaft;
at least one spine comprising a curved proximal portion and a straight distal portion extending along the longitudinal axis;
a first coupler attached to the proximal portion of the at least one spine; and
a second coupler attached to the distal portion of the at least one spine, the second coupler configured to move with respect to the tubular shaft so that movement of the second coupler in a first direction prevents fluid from flowing through the at least one fluid port of the tubular shaft in a first position; and
movement of the second coupler in a second direction permits fluid to flow through the at least one fluid port of the tubular shaft in a second position.

7. The fluid evacuation device of claim 6, the at least one spine configured to slide the second coupler between the first position and the second position.

8. The fluid evacuation device of claim 7, the at least one spine being one of a plurality of spines forming a cage, each of the plurality of spines having a curved proximal portion and a straight distal portion, the curved proximal portion configured to straighten responsive to a retraction of the cage into a sheath.

9. The fluid evacuation device of claim 8, further comprising a sleeve disposed radially around the plurality of spines between the proximal portion and the distal portion, the sleeve attached to the tubular shaft and configured to constrain the curved proximal portion to move relative to the first coupler along the longitudinal axis with respect to the tubular shaft.

10. The fluid evacuation device of claim 8, further comprising a sleeve disposed radially around the plurality of spines between the proximal portion and the distal portion, the sleeve attached to the cage.

11. The fluid evacuation device of claim 10, wherein the tubular shaft comprises a guidewire lumen.

12. The fluid evacuation device of claim 6, the second coupler configured to rotate between the first position and the second position.

13. The fluid evacuation device of claim 12, the at least one spine configured such that a retraction of the at least one spine into a sheath rotates the second coupler from the first position to the second position.

14. The fluid evacuation device of claim 13, the second coupler comprising a hole configured to align with an evacuation port disposed on the tubular shaft when in the second position.

15. A balloon catheter comprising:
a balloon defining an interior volume configured to be inflated by filling with a fluid, the balloon comprising:
a fluid evacuation device comprising:
a tubular shaft extending along a longitudinal axis from a first end to a second end, the tubular shaft including at least one fluid port in fluid communication with a fluid supply to allow fluid flow in and out of the tubular shaft;
at least one spine comprising a curved proximal portion and a straight distal portion extending along a longitudinal axis;
a first coupler coupled to the proximal portion of the at least one spine; and
a second coupler attached to the distal portion of the at least one spine, the second coupler configured to move with respect to the tubular shaft so that movement of the second coupler
prevents fluid from flowing through the at least one fluid port of the tubular shaft in a first position; and
movement of the second coupler permits fluid to flow through the at least one fluid port of the tubular shaft in a second position.

16. The balloon catheter of claim 15, the spine configured to slide the second coupler from the first position to the second position.

17. The balloon catheter of claim 16 further comprising a sheath configured to deliver the balloon, the sheath comprising a distal opening configured to deploy the balloon and receive the balloon upon a retraction of the balloon into the distal opening, the retraction comprising applying a force to the spine and the balloon, and
the spine being one of a plurality of spines forming a cage, each of the plurality of spines having a curved proximal portion and a straight distal portion, the curved portion configured to straighten responsive to a retraction of the cage into a sheath.

18. The balloon catheter of claim 17, the cage configured to slide the second coupler along a tubular shaft from the first position to the second position in response to the force, and the force configured to forcibly deflate the balloon.

19. The balloon catheter of claim 17, the cage configured to rotate the second coupler from the first position to the second position in response to the force, and the force configured to forcibly deflate the balloon.

20. The balloon catheter of claim 15, the balloon comprising a plurality of irrigation ports configured to deliver the fluid.
